Europäisches Patentamt

European Patent Office

Office européen des brevets

(19)

(11) Veröffentlichungsnummer : **0 055 345
B1**

(12) **EUROPÄISCHE PATENTSCHRIFT**

(45) Veröffentlichungstag der Patentschrift :
16.04.86

(21) Anmeldenummer : 81108435.9

(22) Anmeldetag : 16.10.81

(51) Int. Cl.⁴ : **G 01 G  19/44,** G 01 G   3/13,
A 61 B   5/10, G 01 L   1/26

(54) Lagerstatt für einen Patienten mit piezoelektrischen Kraftsignalgeber.

(30) Priorität : 29.12.80 DE 3049347

(43) Veröffentlichungstag der Anmeldung :
07.07.82 Patentblatt 82/27

(45) Bekanntmachung des Hinweises auf die Patenterteilung : 16.04.86 Patentblatt 86/16

(84) Benannte Vertragsstaaten :
AT CH FR GB IT LI

(56) Entgegenhaltungen :
DE-A- 2 700 902
DE-B- 2 852 351
US-A- 3 915 248
Siemens Forschungs- und Entwicklungsberichte,
Band 10, Nr. 2, 1981 Seiten 104-109, Würzburg, DE, H.
REICHENBERGER et al.: "Indirekte Erfassung
mechanischer Lebensfunktionen von Säuglingen"

(73) Patentinhaber : **WALZ ELEKTRONIK GmbH
Walddorfer Strasse 40
D-7271 Rohrdorf (DE)**

(72) Erfinder : **Schneider, Siegfried, Dr. Dipl.-Phys.
Eskilstunastrasse 27
D-8520 Erlangen (DE)**
Erfinder : **Eck, Wolfgang
Leimberger Strasse 15
D-8520 Erlangen (DE)**
Erfinder : **Naser, Georg
Karlstrasse 10
D-8502 Zirndorf (DE)**
Erfinder : **Reichenberger, Helmut, Dr. Dipl.-Phys.
Irisstrasse 8
D-8501 Eckental (DE)**
Erfinder : **Rochling, Hans
Spiegelgraben 41
D-8600 Bamberg (DE)**
Erfinder : **Biller, Max
Erlenweg 8
D-8901 Neusäss 4 (DE)**

(74) Vertreter : **Kinkelin, Ulrich, Dipl.-Ing.
Weimarer Strasse 32/34 Auf dem Goldberg
D-7032 Sindelfingen (DE)**

## Beschreibung

Die Erfindung betrifft eine Lagerstatt für einen Patienten, mit einem Unterteil, einem darauf gelagerten Oberteil und einem dazwischen angeordneten Kraftsignalgeber gemäß Oberbegriff von Anspruch 1.

In der DE-B 28 52 351 ist eine Vorrichtung zur Erfassung von Körperfunktionen von Säuglingen beschrieben, bei der eine Lagerstatt mit einem Unterteil und einem darauf gelagerten Oberteil mit Liegefläche für den Säugling ausgebildet ist, wobei ein piezoelektrischer Kraftsignalgeber mit zwei Keramikscheiben zwischen Ober- und Unterteil angeordnet ist. Der zugehörige Ladungsverstärker ist dabei getrennt angeordnet.

Der Erfindung liegt die Aufgabe zugrunde, die elektrischen Teile bei einer Lagerstatt gemäß Oberbegriff von Anspruch 1 gegen elektrische Störfelder einerseits und gegen mechanische Beanspruchungen, insbesondere gegen Feuchtigkeit, zuverlässig zu schützen ohne daß darunter die Empfindlichkeit leidet.

Die erfindungsgemäße Lösung dieser Aufgabe ist in Anspruch 1 gekennzeichnet. Sie beruht im wesentlichen auf der Zusammenfassung von Kraftsignalgebern und Ladungsverstärkern in einem gemeinsamen, schirmend wirkenden Gehäuse und einer elastischen Hülle, die die Öffnung und den Stempel für die Kraftübertragung abdeckt.

Aus der DE-A 27 00 902 ist zwar ebenfalls ein Kraftsignalgeber mit einem dichten Abschirmgehäuse bekannt. Hierbei ist jedoch der die Kräfte zu dem elektromechanischen Wandler übertragende Stempel in einer flexiblen Membran fest eingespannt. Aus diesem Aufbau resultiert ein erheblicher Aufwand für Fertigung und Justage, zumindest wenn der Geber eine hohe Empfindlichkeit haben soll.

Vorteilhafte Ausgestaltungen der Erfindung sind in den Unteransprüchen gekennzeichnet.

Die Erfindung wird anhand eines in den Figuren dargestellten Ausführungsbeispieles näher erläutert ; es zeigen

Figuren 1 und 2  in schematischer Darstellung eine Vorrichtung zur Überwachung von Lebensfunktionen von Säuglingen, bei der ein erfindungsgemäßer Kraftsignalgeber eingesetzt ist,

Figuren 3 bis 5  den darin verwendeten Kraftsignalgeber in seinem Aufbau,

Figur 6  die elektrische Schaltung des vorher dargestellten Kraftsignalgebers.

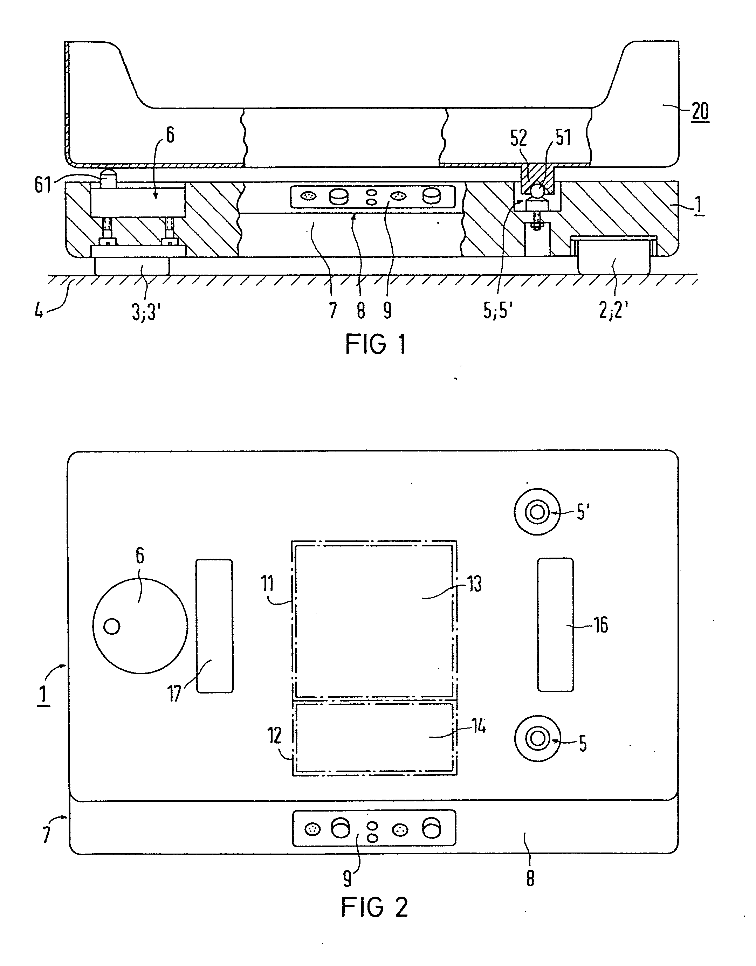

In den Fig. 1 und 2 bedeuten 1 das Unterteil und 20 das Oberteil mit Liegefläche einer Vorrichtung zur Erfassung von Körperfunktionen von Säuglingen. Mit den Bezugszeichen 2 bzw. 2′ und 3 bzw. 3′ sind separate Dämpfungskörper im Unterteil 1 bezeichnet, mit denen das Unterteil auf einer Stellfläche 4, beispielsweise in einem Inkubator, aufliegt. Mit 5 bzw. 5′ sind zwei erste mechanische Lager und mit 6 ein drittes, als Kraftsignalgeber ausgebildetes Lager bezeichnet. Die drei Lagerpunkte bilden in der Aufsicht ein gleichschenkeliges Dreieck. Über den Kraftsignalgeber 6 werden die Lebensfunktionen des Säuglings aufgenommen und einer Einheit zur Meßwertverarbeitung zugeführt. Die Vitalwerte können dann als optische und/oder akustische Signale an einer Anzeigeeinheit 9, die an einem Seitenteil 7 mit schräg ausgebildeter Fläche 8 des Unterteils 1 angebracht ist, angezeigt werden.

Die Lager 5 bzw. 5′ sind als stabile Bolzenlager ausgeführt und bestehen im einzelnen aus einer auf einem Stift angeordneten Kugel 51, die in einem Gegenstück 52 des Oberteils 20 mit kegelförmiger Aussparung eingreift. Es ergibt sich so bei Abnehmen und anschließendem Wiederaufsetzen des Oberteils 20 immer eine exakte Lagerstellung auf dem Unterteil 1. Der Kraftsignalgeber 6 ist als eine geschlossene Gehäuseeinheit dargestellt, die im Unterteil 1 fest verschraubt ist und von der als Auflagepunkt für die Krafteinleitung ein kalottenförmiger Stempel 61 herausragt. Es wird dadurch ein punktförmiges Angreifen der zu erfassenden Kräfte sichergestellt und ein entsprechendes elektrisches Signal gewonnen.

Das Unterteil 1 bildet insgesamt einen quaderförmigen Flachkörper und weist zur Handhabung Durchbrüche 16 und 17 auf. Im Unterteil 1 befinden sich weiterhin in der Fig. 2 gestrichelt angedeutete Aussparungen 11 und 12. In diese Aussparungen können einzelne Einheiten 13 und 14 zur Energieversorgung und Signalverarbeitung, die auf die Raumform der Aussparungen abgestimmt sind, eingeschoben werden. Dabei ist es günstig, einzelne Module vorzusehen. Es kann so beispielsweise die Einheit 13 für die Energieversorgung als aufladbarer Akkumulator vorhanden sein, während eine Einheit 14 zur Signalverarbeitung entweder als fest verdrahtete Schaltung oder aber auch als Rechnereinheit zur software-mäßigen Auswertung der Signale vorgesehen ist. Von der Signalverarbeitungseinheit 14 wird die frontseitige Anzeigeeinheit 9 mit optischen und akustischen Elementen und einem Signalausgang angesteuert.

Das Oberteil 20 besteht im wesentlichen aus einer Schale aus schlagzähem Polyurethan-Integral-Hartschaum mit einer Liegefläche für den Säugling.

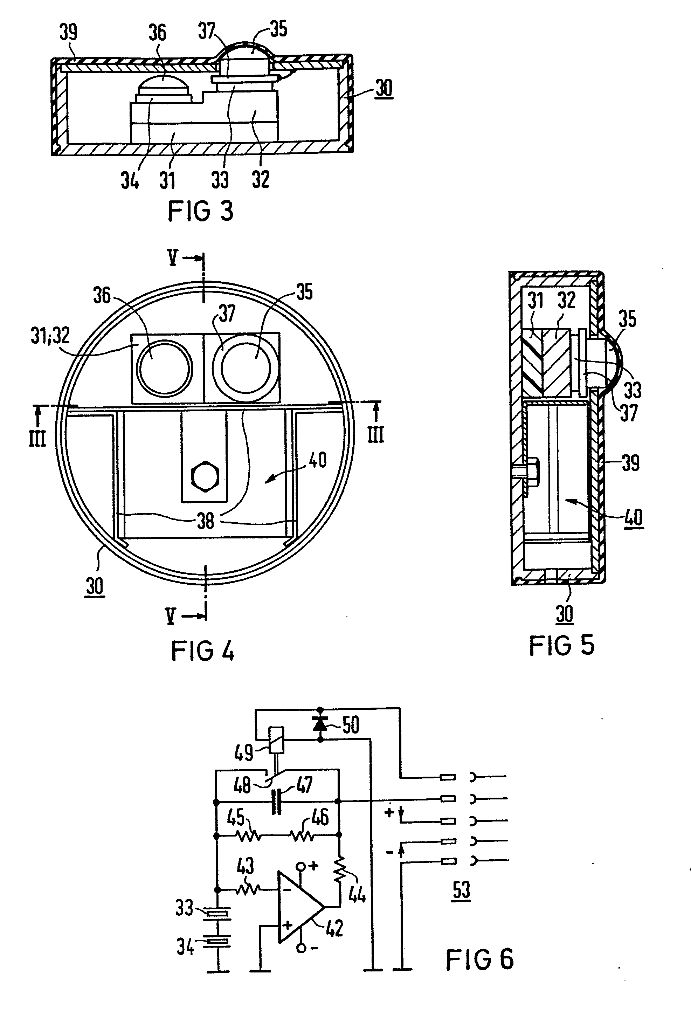

In den Fig. 3 bis 5 kennzeichnet 30 ein metallisches Gehäuse, das alle Elemente und Bauteile des erfindungsgemäßen Kraftsignalgebers aufnimmt und dem Kraftsignalgeber 6 der Fig. 1 und 2 entspricht. Das Gehäuse 30 ist als flaches, hohlzylinderförmiges Bauteil mit kreisförmiger Grundfläche ausgebildet und besteht beispielsweise aus Aluminium. Das Gehäuse 30 ist also gegen äußere elektrische Einflüsse abgeschirmt und bildet in seinem Inneren durch weitere metallische Abschirmungen ein erstes Abteil für einen Kraftaufnehmer und ein zweites Abteil für einen Ladungsverstärker. Der gesamte Innenraum des Gehäuses ist zusammen mit den Bauteilen in Kunststoff vergossen. Der räumliche Aufbau dieser Teile ist nachfolgend im einzelnen beschrieben :

Ein Kraftaufnehmer und ein entsprechendes Kompensationselement sind zusammen auf einem gemeinsamen Sockel angeordnet, der gegen den Boden des Gehäuses 30 isoliert ist. Dabei weisen Kraftaufnehmer und Kompensations-element etwa die gleiche Geometrie auf mit dem Unterschied, daß eine Krafteinleitung lediglich zum Kraftaufnehmer erfolgen kann. Es hat sich als günstig erwiesen, zur Isolation gegen das Gehäuse 30 den Sockel zu unterteilen in einen quaderförmigen Kunststoffblock 31, z. B. aus Polyacetal, und einen darüber bündig angeordneten Metallblock 32, z. B. aus Aluminium, der eine Stufe aufweist. Bei solcher Ausbildung des Sockels können Kraftaufnehmer und Kompensationselement im einzelnen weitgehend gleich aufgebaut sein, wobei der Kraftaufnehmer auf dem höheren Niveau und das Kompensationselement auf dem niedrigeren Niveau des aus den Blöcken 31 und 32 gebildeten Sockels sitzt.

Im einzelnen sind auf dem Block 32 zwei gleichgroße Scheiben 33 und 34 aus piezoelektrischem Keramikmaterial angeordnet, beispielsweise aus Bleizirkonattitanat. Auf den Scheiben 33 und 34 befinden sich jeweils Stempel 35 und 36 mit kugelförmiger Oberfläche. Dabei bildet der Stempel 35 ein Koppelelement (entsprechend dem Stempel 61 in Fig 1) für die Krafteinleitung und ist so hoch, daß seine Oberfläche aus einer Öffnung des Gehäuses 30 herausragt, während der Stempel 36 des Kompensationselementes mit seiner Oberfläche unbelastet innerhalb des Gehäuses 30 liegt. Zwischen Stempel 35 und Scheibe 33 des Signalaufnehmers ist noch eine Scheibe 37 aus elektrisch leitfähigem Material, beispielsweise Kupfer, zur zusätzlichen Abschirmung angebracht. Die Abmessungen der Scheibe 37 entsprechen mindestens der Öffnung im Gehäuse 30 ; Scheibe 37 und Gehäuse 30 sind elektrisch leitend verbunden.

Auf das Gehäuse 30 ist eine Hülle 39 aus Elastomer aufgezogen, die mit ihrem äußeren Rand in eine am Gehäuseboden umlaufende Nut eingreift. Im Bereich der Öffnung des Gehäuses 30 ist die Hülle 39 als dünne Membran ausgebildet und liegt auf dem Stempel 35 lose auf. Die Krafteinleitung auf den Kraftaufnehmer wird also nicht beeinflußt ; trotzdem ist ein dichter Abschluß gewährleistet, so daß Feuchtigkeit und dergleichen nicht in das Gehäuse eindringen kann.

Das erste Abteil des Gehäuses 30 mit dem Kraftaufnehmer und Kompensationselement ist vom zweiten Abteil durch Abschirmbleche 38 abgeteilt, die wiederum aus Aluminium bestehen können. Das so gebildete beispielsweise quaderförmige Volumen ist dadurch vollständig metallisch eingeschlossen und nimmt einen Ladungsverstärker 40 auf.

In Fig. 6 sind die beiden piezokeramischen Scheiben 33, 34 aus Fig 3 bis 5 bezüglich ihrer Polarisation gegensinnig hintereinandergeschaltet und an einen Operationsverstärker 42 angeschlossen, der mit Widerständen 43 bis 46 und einem Rückkoppelkondensator 47 beschaltet ist und so den Ladungsverstärker 40 bildet. Parallel zum Rückkoppelkondensator 47 liegt eine Kurzschlußleitung mit einem Schalter 48. Dieser Schalter wird durch ein Relais 49 mit zugehöriger Sperrdiode 50 dann geschlossen, wenn die Ladung am Ladungsverstärker einen Grenzwert überschreitet. Mit 53 ist ein Anschluß bezeichnet, über den der Kraftsignalgeber mit einer Einheit zur Signalverarbeitung, die in Fig 2 mit 14 bezeichnet wurde, verbunden werden kann.

**Patentansprüche**

1. Lagerstatt für einen Patienten, mit einem Unterteil (1), einem darauf gelagerten Oberteil (20) und einem dazwischen angeordneten Kraftsignalgeber (6) mit einer ersten piezoelektrischen Scheibe (33), an die ein Ladungsverstärker (40) angeschlossen ist, auf der sich das Oberteil (20) über einen Stempel (35) als Koppelelement mechanisch abstützt, und mit einer zweiten piezoelektrischen Scheibe (34) als Kompensationselement, das in einer gehäuseartigen Ausnehmung des Unterteiles (1) angeordnet ist, dadurch gekennzeichnet,
daß die piezoelektrischen Scheiben (33, 34) und der Ladungsverstärker (40) in einem gemeinsamen geschlossenen Gehäuse (30) aus Metall untergebracht sind, das im Unterteil (1) der Lagerstatt angeordnet ist,
daß die erste piezoelektrische Scheibe (33) zwischen dem Boden des Gehäuses (30) und dem Stempel (35) angeordnet ist,
daß das Gehäuse (30) eine Öffnung aufweist, durch die der Stempel (35) mit einer Kugelfläche nach außen ragt,
und daß die Öffnung des Gehäuses durch eine lose auf dem Stempel (35) aufliegende, elastische Hülle (39) in Form einer dünnen Membran flüssigkeitsdicht abgeschlossen ist.

2. Lagerstatt nach Anspruch 1, dadurch gekennzeichnet, daß die Hülle (39) aus Elastomer und auf das Gehäuse (30) aufsteckbar ist.

3. Lagerstatt nach Anspruch 1 oder 2, dadurch gekennzeichnet, daß zwischen dem Stempel (35) und der piezoelektrischen Scheibe (33) eine Scheibe (37) aus elektrisch leitendem Material angeordnet und mit dem Gehäuse elektrisch leitend verbunden ist, und daß der Durchmesser der Scheibe mindestens dem Durchmesser der Öffnung des Gehäuses gleich ist.

4. Lagerstatt nach Anspruch 3, dadurch gekennzeichnet, daß der Ladungsverstärker (40) innerhalb des Gehäuses (30) zusätzlich von einem elektrisch leitenden Schirm (38) umgeben ist.

5. Lagerstatt nach einem der Ansprüche 1 bis 4, dadurch gekennzeichnet, daß die beiden piezoelektrischen Scheiben (33, 34) auf einem Sockel gegenüber dem Gehäuse (30) isoliert angeordnet und gegensinnig in Reihenschaltung in den Eingangskreis des Ladungsverstärkers (40) geschaltet sind.

6. Lagerstatt nach Anspruch 5, dadurch gekennzeichnet, daß der Sockel aus einem mit dem Gehäuse (30) verbundenen Kunststoffblock (31) und einem darauf befestigten Metallblock (32) besteht und die Scheiben (33, 34) auf dem Metallblock befestigt sind.

7. Lagerstatt nach Anspruch 6, dadurch gekennzeichnet, daß der Metallblock (32) stufenförmig ausgebildet und der Kraftaufnehmer auf der höheren und das Kompensationselement auf der niedrigeren Stufe angeordnet ist.

8. Lagerstatt nach Anspruch 7, dadurch gekennzeichnet, daß die piezoelektrische Scheibe (34) des Kompensationselementes zwischen dem Metallblock und einer Elektrode angeordnet ist, deren Form und Material dem Stempel des Kraftaufnehmers entspricht.

9. Lagerstatt nach einem der Ansprüche 1 bis 8, dadurch gekennzeichnet, daß die piezoelektrischen Scheiben (33, 34) mit ihrem Sockel einerseits und der Ladungsverstärker (42) mit seinen Beschaltungselementen andererseits in dem Gehäuse in Kunststoff eingegossen sind.

10. Lagerstatt nach einem der Ansprüche 1 bis 8, dadurch gekennzeichnet, daß das Gehäuse (30) als flacher Hohlzylinder mit Kreisquerschnitt ausgebildet ist.

## Claims

1. A bed for a patient, comprising a lower part (1), an upper part (20) arranged upon the lower part, and a force signal generator (6) arranged therebetween and having a first piezoelectric disc (33) connected to a charge amplifier (40), upon which the upper part (20) is mechanically supported through a prop (35) serving as a coupling element, and having a second piezoelectric disc (34) serving as a compensation element, arranged in a housing-like recess in the lower part (1), characterised in

that the piezoelectric discs (33, 34) and the charge amplifier (40) are accommodated in a common closed metal housing (30) which is arranged in the lower part (1) of the bed,

that the first piezoelectric disc (33) is arranged between the base of the housing (30) and the prop (35),

that the housing (30) has an opening through which a conical surface of the prop (35) projects outwards,

and that the opening in the housing is sealed so as to be impervious to liquids by means of an elastic sleeve in the form of a thin membrane (39), which rests slackly on the prop (35).

2. A bed as claimed in Claim 1, characterised in that the sleeve (39) consists of elastomer and can be attached to the housing (30).

3. A bed as claimed in Claim 1 or Claim 2, characterised in that a disc (37) of electrically conductive material is arranged between the prop (35) and the piezoelectric disc (33) and is electrically conductively connected to the housing ; and that the diameter of the disc is at least equal to the diameter of the opening in the housing.

4. A bed as claimed in Claim 3, characterised in that the charge amplifier (40) within the housing (30) is additionally surrounded by an electrically conductive screen (38).

5. A bed as claimed in one of Claims 1 to 4, characterised in that the two piezoelectric discs (33, 34) are arranged on a base so as to be insulated from the housing (30) and are connected in series in opposite directions in the input circuit of the charge amplifier (40).

6. A bed as claimed in Claim 5, characterised in that the base consists of a synthetic resin block (31) connected to the housing (30) and a metal block (32) attached thereto, and the discs (33, 34) are attached to the metal block.

7. A bed as claimed in Claim 6, characterised in that the metal block (32) is of stepped formation, the force pick-up being arranged on the higher step, whilst the compensation element is arranged on the lower step.

8. A bed as claimed in Claim 7, characterised in that the piezoelectric disc (34) of the compensation element is arranged between the metal block and an electrode, the shape and material of which correspond to the prop of the force pick-up.

9. A bed as claimed in one of Claims 1 to 8, characterised in that the piezoelectric discs (33, 34) with their base, on the one hand, and the charge amplifier (42) with its wiring elements, on the other hand, are cast in synthetic resin in the housing.

10. A bed as claimed in one of Claims 1 to 8, characterised in that the housing (30) is designed as a smooth hollow cylinder of circular cross-section.

## Revendications

1. Lit pour patients, comportant une partie inférieure (1) une partie supérieure (20) montée sur la précédente et un générateur de signaux de force (6) disposé entre ces deux parties, pourvu d'un premier disque piézoélectrique (33) auquel est relié un amplificateur de charge (40) et sur lequel prend appui mécaniquement, la partie supérieure (20) par l'intermédiaire d'un poussoir (35) servant l'élément de couplage, ainsi que d'un second disque piézoélectrique (34) faisant office d'élément de compensation et disposé dans une cavité, en forme de boîtier, ménagée dans la partie inférieure, caractérisé par le fait,

que les disques piézoélectriques (33, 34) et l'amplificateur de charge (40) sont logés dans un boîtier métallique commun (30) qui est disposé dans la partie inférieure du lit (1),

que le premier disque piézoélectrique (33) est disposé entre le fond du boîtier (30) et le poussoir (35),

que le boîtier (30) est pourvu d'une ouverture à travers laquelle émerge vers l'extérieur le poussoir (35) par une surface sphérique,

et que l'ouverture du boîtier est fermée de façon étanche à un liquide, à l'aide d'une enveloppe élastique (39) de la forme d'une membrane mince reposant librement sur le poussoir (35).

2. Lit selon la revendication 1, caractérisé par le fait que l'enveloppe (39) est en un élastomère et est enfichable sur le boîtier (30).

3. Lit selon la revendication 1 ou 2, caractérisé par le fait qu'entre le poussoir (35) et le disque piézoélectrique (33) est disposé un disque (37) en un matériau électriquement conducteur et relié électriquement au boîtier, et que le diamètre du disque est au moins égal au diamètre de l'ouverture du boîtier.

4. Lit selon la revendication 3, caractérisé par le fait que l'amplificateur de charge (40) est de plus entouré à l'intérieur du boîtier (30) par un écran électriquement conducteur (38).

5. Lit selon l'une des revendications 1 à 4, caractérisé par le fait que les deux disques piézoélectriques (33, 34) sont disposés avec isolation par rapport au boîtier (30) sur un socle, et ils sont montés en sens inverse dans un circuit série du circuit d'entrée de l'amplificateur de charge (40).

6. Lit selon la revendication 5, caractérisé par le fait que le socle est constitué par un bloc de matière plastique (31) qui est relié au boîtier (30) et par un bloc métallique (32) fixé sur le bloc en matière plastique, et les deux disques (30, 34) sont fixés sur le bloc métallique.

7. Lit selon la revendication 6, caractérisé par le fait que le bloc métallique (32) est réalisé avec des paliers et le capteur de force est disposé sur l'étage le plus haut alors que l'élément de compensation est disposé sur l'étage le plus bas.

8. Lit selon la revendication 7, caractérisé par le fait que le disque piézoélectrique (34) de l'élément de compensation est disposé entre le bloc métallique et une électrode dont la forme et le matériau correspondent au poussoir du capteur de force.

9. Lit selon l'une des revendications 1 à 8, caractérisé par le fait que les disques piézoélectriques (33, 34) et leur socle, d'une part, et l'amplificateur de charge (42) et ses éléments de circuit, d'autre part, sont noyés, dans le boîtier, avec une matière plastique.

10. Lit selon l'une des revendications 1 à 8, caractérisé par le fait que le boîtier (30) est réalisé sous la forme d'un cylindre creux et plat de section transversale circulaire.

FIG 1

FIG 2

FIG 3

FIG 4

FIG 5

FIG 6